# EUROPEAN PATENT SPECIFICATION

(11) **EP 0 623 030 B1**
(45) Date of publication and mention of the grant of the patent: **19.03.1997**
(21) Application number: 92925467.0
(22) Date of filing: 01.12.1992
(51) Int. Cl.: A61L 17/00, A61L 27/00, A61L 31/00, C08G 69/44

(54) **BIOABSORBABLE POLY(ESTERAMIDE) AND METHOD FOR MAKING SAME**
Bioabsorbierbares Polyesteramid und Verfahren zur Herstellung
POLY(ESTERAMIDE) BIOABSORBABLE ET METHODE DE FABRICATION

(30) Priority: 15.01.1992 US 821024; 07.08.1992 US 927447
(43) Date of publication of application: 09.11.1994
(73) Proprietor: MINNESOTA MINING AND MANUFACTURING COMPANY, St. Paul, Minnesota 55133-3427 (US)
(72) Inventor: BARROWS, Thomas, H., Saint Paul, MN 55133-3427 (US); TRUONG, Myhanh, T., Saint Paul, MN 55133-3427 (US)
(74) Representative: Ahner, Francis
(86) International application number: US9210352
(87) International publication number: WO9313814

(56) References cited:
- EP-A- 0 030 822
- US-A- 4 209 607
- US-A- 4 226 243

## Description

### Field of Invention

The present invention relates to bioabsorbable polymers, particularly poly(esteramides).

### Background of the Invention

Poly(esteramides) are known to be useful bioabsorbable polymeric materials derived from reacting diamidediols with dicarboxylic acids, derivatives thereof or bischloroformates. Such polymers and some of their uses are described in U.S. Patent Nos. 4,343,931; 4,529,792; 4,534,349; 4,669,474; 4,719,917; 4,883,618; and 5,013,315 (all Barrows et al.).

An increasing number of surgically implantable devices that only function for a relatively short period of time in vivo are being designed from synthetic polymers that are eliminated from the body by hydrolytic degradation and subsequent metabolism after serving their intended purpose. The molecular weight of such "bioabsorbable" polymers is an important parameter in determining whether or not a particular lot of polymer will perform properly for an adequate length of time in any specific application.

Thus, if significant loss of molecular weight occurs during processing of the polymer into the device, such as by melt extrusion, then the device may fail prematurely in vivo as hydrolysis shifts the molecular weight distribution below a minimum range required for the device to function properly. Loss of molecular weight upon extrusion is commonly encountered with polyesters even when moisture in the resin has been reduced to the lowest possible level. For example, poly-L-lactide, a well known bioabsorbable polyester has not yet been melt extruded into fiber with molecular weight much higher than 100,000 since the extrusion process degrades higher molecular weight fractions to this relatively constant maximum value.

Bioabsorbable poly(esteramides) described in U.S. Patent Nos. 4,343,931 and 4,529,792 offer an advantage over poly-L-lactide and related polyesters in that a lower molecular weight is adequate to achieve comparable fiber strength due to the intermolecular hydrogen bonding provided by the amide linkages. Fibers made of such polymers exhibit comparable strength to those made of poly-L-lactide while providing lower modulus (and thus greater flexibility) and greater toughness and durability. Compared to poly-L-lactide they also are more rapidly bioabsorbed.

### Summary of Invention

It has been discovered that a certain class of poly(esteramides) provides surprisingly superior results in comparison to other poly(esteramides). In particular, the poly(esteramides) of the invention provide a heretofore unrecognized combination of rapid degradation and metabolic absorption in vivo and long term strength maintenance. The invention also provides a novel method for making such polymers. The invention further provides bioabsorbable fibers and other implantable devices prepared from such poly(esteramides) and offering advantageous combinations of properties.

In brief summary, the bioabsorbable poly(esteramides) of the invention comprise a plurality of units of the general formula: 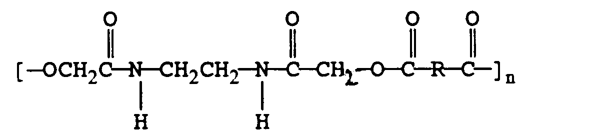 wherein:
a) R is selected from the group consisting of linear or branched alkylenes having from 2 to 25 carbon atoms;
b) n has an average value between about 20 and about 1000.

Implantable devices, e.g., fibers, made of the poly(esteramides) provided herein offer the combination of superior strength retention and superior flex life with rapid metabolic absorption as compared to previous poly(esteramides). This invention also relates to an improved process for preparing poly(esteramide) polymers with exceptional fiber-forming potential.

Surprisingly, the polymers produced by the process of the invention exhibit superior fiber-forming potential as compared to poly(esteramides) produced by conventional processes.

Briefly summarizing, the novel process of the invention comprises:
a) reacting ethylenediamine with glycolic acid to form a diamidediol; and
b) reacting the diamidediol with one or more diacid chlorides;
to yield a poly(esteramide) polymer of the invention.

The novel poly(esteramides) of the invention are particularly useful for use in fibers and implantable devices incorporating fibers, e.g., surgical sutures and bioabsorbable scaffolds for tissue regeneration such as ligament augmentation devices in mammals.

To indicate the number of carbon atoms in the backbones of the diamine functionality and the diacid functionality for the case of linear aliphatic alkylenes, an abbreviated designation in the form "PEA-x,y" has been coined, and is used in some places within this application. In the designation, "x" refers to the number of carbon atoms exclusive of the carbonyl carbon atoms in the backbone of the diacid functionality and "y" refers to the number of carbon atoms in the backbone of the diamine functionality. For example, poly[decane-1,10-di(carbonyloxy)ethane-1,2- di(amidocarbonylmethylene)], a poly(esteramide) polymer of the invention, whose synthesis is exemplified in Example 1 below would be referred to as PEA-10,2.

### Detailed Description of Illustrative Embodiments

The poly(esteramides) of the present invention are polymers having a plurality of units of the general formula: 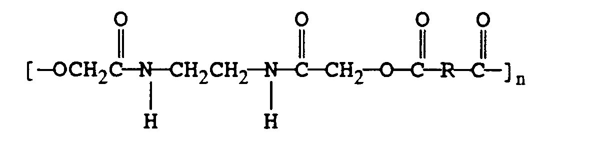 wherein:
a) R is selected from the group consisting of linear or branched alkylenes having from 2 to 25 carbon atoms; and
b) n has an average value from about 20 to about 1000.

As a result of the hydrogen atoms on the nitrogen atoms of the amine groups, polymers of the invention tend to form hydrogen bonds, thereby affording nylon-like flexibility and strength properties.

Polymers of the invention where R is a linear alkylene of 2 to 14 carbon atoms are presently preferred, and most preferred is a linear alkylene of 4 to 12 carbon atoms or R is a linear alkylene having 10 carbon atoms; or R is a linear alkylene having 6 carbon atoms. Polymers of the invention wherein R has an even number of carbon atoms are typically preferred because starting materials are generally more accessible than those for compounds wherein R has an odd number of carbon atoms.

Polymers of the invention wherein n is 20 to 1000 are presently preferred because such polymers provide a more generally useful combination of strength and rate of in vivo absorption. Most preferred are polymers wherein n is about 100 to 400 because such polymers provide what is generally an optimal balance of strength and rate of in vivo absorption. Polymers with relatively lower molecular weight (i.e., relatively smaller n) provide lower strength with faster absorption whereas polymers with relatively higher molecular weight (i.e., relatively larger n) provide greater strength with slower absorption.

It has been found that polymers (and fibers formed therefrom) based on forming diamidediols from 1,2-ethanediamine and glycolic acid possess the advantageous properties found in this invention to a surprising degree.

In order to obtain the regular sequence shown in the above polymers, it is typically desirable to first form the amide linkages prior to polymerization. This can be accomplished by combining about two moles of glycolic acid with about one mole of the diamine and heating at a temperature between 150° to 220°C until distillation of water is complete. Alternatively, combination of hydroxy acid and diamine will produce a salt which can be purified by recrystallization and then subjected to the above condensation. In either case, a high yield of diamidediol is obtained which can be purified by recrystallization.

Synthesis of the preferred polymers of the invention with an inherent viscosity suitable to obtain fibers with adequate strength is preferably carried out as described herein. The preferred process for forming poly(esteramides) having a suitably high molecular weight (i.e., n is 20 or more) comprises:
a) suspending diamidediol, preferably in powder form, in a liquid (referred to herein as a "synthesis solvent") which: 1) is a nonreactive solvent for acid chlorides, 2) is a nonsolvent for diamidediols, 3) has a boiling point of 100°C or higher, and 4) is preferably substantially free of water;
b) adding a stoichiometrically equal amount of one or more diacid chlorides;
c) heating the mixture to a moderate temperature, e.g., 60° to 90°C, until low molecular weight, i.e., an inherent viscosity of between about 0.3 and about 0.6, is achieved; and then
d) rapidly refluxing with vigorous mechanical mixing (e.g. motor driven paddle), typically until the polymer has an inherent viscosity of at least 1.0, preferably about 1.2 to about 1.5;
to yield poly(esteramide) of the desired formula. By "nonsolvent" it is meant that the subject liquid, at its boiling point, will not solvate more than 2 weight percent of the subject material. Preferably, the liquid will solvate substantially none of the subject material. By "nonreactive" it is meant that the subject liquid will not react with any of the species (precursors, intermediates, and reaction products) of the subject reaction.

Preferably the inherent viscosity of the polymer is frequently or continuously monitored throughout the synthesis. As used herein, inherent viscosity is measured at 30°C in 2,2,2-trifluoroethanol. If the synthesis is halted while the inherent viscosity is still at too low a level, the molecular weight of the resultant polymer will be too low and it will exhibit poor strength and tend to be brittle; whereas if the synthesis is not halted soon enough and the inherent viscosity reaches too high a level, e.g., about 1.5 to 1.7 or more, the resultant polymer may gel, at least in some portions. Gelled portions typically lead to weak points in fibers made from such polymers. It has been observed that subsequent processing of poly(esteramides) to fabricate implants, typically involving thermal processing, tends to reduce the inherent viscosity of the polymer.

Illustrative examples of suitable synthesis solvents include methyl chloroacetate, high boiling ketones, toluene, chlorobenzene, xylene, 1,1,2-trichloroethane, and 1,4-dioxane. The preferred solvents for use with a diacid chloride are chlorobenzene and toluene because of their useful boiling points. Toluene is most preferred because of its generally held status as a toxicologically safe agent. If desired, mixtures of synthesis solvents may be used. Typically, solvents and solvent mixtures which have boiling points (at 1 atmosphere) of between about 70°C and about 150°C, preferably between about 90°C and about 120°C, are useful.

The preferred synthesis method has the advantages of not requiring catalyst, of yielding product in a relatively short period of time, and of producing high molecular weight polymer, typically free of crosslinking in a powder or granular, easy-to-manipulate form. In addition, moisture which would otherwise react with the acid chloride can be readily excluded from the system by azeotropic distillation prior to addition of the diacid chloride.

Dicarboxylic acid chlorides useful in the synthesis of polymers by the above methods include those derived from dicarboxylic acids listed below. Illustrative examples of suitable dicarboxylic acids include: oxalic acid; malonic acid, succinic acid; 2,3-dimethylsuccinic acid; glutaric acid,; 3,3-dimethylglutaric acid; 3-methyladipic acid; adipic acid; pimelic acid; suberic acid; azelaic acid; sebacic acid; 1,9-nonanedicarboxylic acid; 1,10-decanedicarboxylic acid; 1,11-undecanedicarboxylic acid; 1,12-(s)dodecanedicarboxylic acid; 1,13-tridecanedicarboxylic acid; 1,14-tetradecanedicarboxylic acid; 1,15-pentadecanedicarboxylic acid; and 1,16-hexadecanedicarboxylic acid. Linear diacids containing two to twelve -CH₂- groups are preferred as they degrade into agents known to be metabolically satisfactory. The longer members of this class are typically preferred for applications wherein the resultant polymer is to be used in ligament augmentation devices and other devices wherein relatively long performance is required, e.g., about six months or so. The shorter members of this class are typically preferred for applications wherein the resultant polymer is to be used in devices such as sutures from which shorter performance, e.g., about two to three months, is desired. In the presently most preferred polymer, R is preferably decane, e.g., formed by the removal of the chloride from dodecanedioyl chloride. It is highly preferred that the acid chlorides be carefully purified, e.g., by fractional distillation. Mixtures of dicarboxylic acid chlorides can be reacted with the diamidediol to provide polymers wherein R is varied. Such copolymers may be used to optimize desirable combinations of properties, e.g., strength and flexibility.

Although the poly(esteramides) described herein can provide previously unattained combinations of strength retention and rapid metabolic absorption, it is preferred that they be treated with an amide group-containing solvent after synthesis. To begin the treatment, the polymer must be suspended in a non-reactive liquid medium (for which the previously mentioned synthesis solvents are suitable). Accordingly, if the poly(esteramide) polymer was dried following synthesis, it must be suspended in the liquid medium. If it is still suspended in the synthesis solvent, it may be treated as follows directly. The polymer is dissolved or extracted with an amide group-containing solvent, typically while heating and stirring. After it is fully dissolved and well mixed, the poly(esteramide) is precipitated from the resultant solution, e.g., by cooling. The solvent is then removed, e.g., by filtering or centrifuging out the precipitated polymer, decanting, etc. Substantially all of the synthesis residues, e.g., species containing acid chloride functionalities, remain dissolved in the amide group-containing solvent. Typically, the liquid medium is then removed, e.g., by vacuum and tumble drying. It is typically preferred to treat the poly(esteramide) within a week of its synthesis, more preferably immediately after its synthesis to avoid degradation during storage. If necessary to delay treatment for a time after synthesis, removal of the synthesis solvent is generally preferred to reduce degradation during storage. An advantage of the treatment process is that typically the poly(esteramide) can be treated in the same vessel it was synthesized in, typically using the synthesis solvent as the liquid medium. Poly(esteramides) which have been treated exhibit improved storage and heat stability, thereby increasing their shelf life as virgin polymer and in fabricated implantable devices as well as improving their tolerance for melt processing.

Illustrative examples of amide-group containing solvents suitable for use in the treatment process include N-methylpyrrolidone, N-methylformamide, N-methylacetamide, N,N-dimethylacetamide, formamide, N,N-dimethylformamide, tetramethylurea, and the like. Tetramethylurea and N-methylpyrrolidone are preferred, with N-methylpyrrolidone being most preferred, because these solvents are known as toxicologically safe compounds. Typically, poly(esteramides) treated in accordance with the invention will retain small quantities of the amide group-containing solvent, e.g., typically about 1.0 weight percent or less, sometimes about 0.05 weight percent or less.

The ratio of liquid medium and amide group-containing solvent is typically preferably about 1:1. It will be understood that other ratios of liquid medium and solvent may be used in accordance with the present invention. Amide group-containing solvents are typically very good solvents for poly(esteramides) but may contain trace amounts of moisture. Because the moisture may lead to degradation of the polymer, thereby tending to limit the in vivo performance of a device incorporating the polymer, it is typically desirable to minimize how much of the solvent is used, using only enough to fully dissolve the poly(esteramide). Minimizing the amount of solvent used also facilitates precipitation of the polymer from the solvent and removal of the solvent as well.

The solution is heated, typically while stirring, to the boiling point of the liquid medium, to facilitate dissolving the poly(esteramide). Once the polymer is fully dissolved, it then is precipitated from solution, e.g., by allowing the solution to cool slowly; adding additional amounts of liquid medium, preferably at the same temperature as the solution; and/or pouring the solution into liquid medium (perhaps a different liquid medium than was used for suspension). To avoid agglomeration of polymer and obtain more uniform treatment, stirring speed may be increased. Small amounts of hot liquid medium may be added to control or temper the rate of precipitation and prevent coagulation of polymer.

The polymeric materials of this invention can be fabricated into films and fibers by melt extrusion. When the polymer is fabricated into fibers, it is typically preferred that n of the general formula have an average value from about 100 to about 400. Such fibers have been implanted subcutaneously in rats and have been found to be non-irritating and compatible with the living tissue over the time span of many months.

The poly(esteramides) of the invention provide a combination of superior strength retention and more rapid metabolic absorption than do previous poly(esteramides). For instance, melt-spun fiber made from poly[decane-1,10-di(carbonyloxy)ethane-1,2-di(amidocarbonylmethylene)], (i.e., PEA-10, 2) a polymer of the invention, has been observed to retain 80 percent of its initial tensile strength after three months, 50 percent of its initial tensile strength after six months, and 20 percent of its initial tensile strength after more than nine months residence in a solution of pH 7.4 held at 37°C to simulate in vivo implant conditions. Contrarily, melt-spun fiber made from poly(oxysuccinoyloxydodecane-1,12-di(amidocarbonylmethylene)), (i.e., PEA-2, 12) a polymer which is structurally similar to the above polymer except that it is made from a long methylene chain diamine and a short chain diacid rather than a short chain diamine and a long chain diacid, has been observed to retain only 20 percent of its initial strength after three months residence as an in vivo implant in mice. In view of this difference in strength retention one skilled in the art would assume that the fiber exhibiting longer strength retention would also take longer to hydrolyze. However, as shown in Comparative Example A, this fiber was surprisingly found to hydrolyze faster than the comparative fiber of the prior art which had faster strength loss.

The polymers of the present invention are also useful in the manufacture of cast and/or extruded films and molded solid surgical acids. Thus, cylindrical pins, screws, reinforcing plates, etc. may be machined from the cast or molded polymer having the aforementioned in vivo absorption characteristics.

In order to evaluate thermal stability, the polymers were melted using an Instron Model 4202 Melt Rheometer. A 10 gram sample was used each time and each sample was held for 10 minutes in the rheometer. Inherent viscosity was determined after each melting and the data were recorded. Melt samples of poly(esteramides) at elevated temperatures with and without precipitation by N-methylpyrrolidone ("NMP") were analyzed for molecular weight distribution using gel permeation chromatography (GPC). It has been found from such data that the treatment of poly(esteramides) of the invention with NMP results in a significant improvement in retention of molecular weight after melt processing in the temperature range of typical utility (i.e., 170° to 180°C). It is believed that all related poly(esteramide) extrusion resins will similarly benefit from this type of treatment with NMP or other such solvents.

### Examples

The invention will be further explained by the following illustrative examples.

### Example 1

### Synthesis and treatment of poly[decane-1,10-di(carbonyloxy)ethane-1,2-di(amidocarbonylmethylene)] (i.e., PEA-10,2).

Exactly 471.63 grams ("g") of 1,2-di(hydroxyacetamido)ethane were mixed well with 1.5 liters ("l") of dry toluene and placed in a 5 l three neck flask. To this mixture was added 2 l of dry toluene (less than 50 ppm water) and then 715.26 g of distilled dodecanedioyl chloride. The mixture was heated and stirred under nitrogen at 90° to 95°C for six hours, then at reflux for about two hours. About 1.5 l of hot toluene were added. The inherent viscosity of the reacting mixture was measured periodically until it reached 1.2, then the reaction product was filtered and dried in a vacuum oven tumble drier for about 2 hours.

The dry polymer powder was placed in a 22 l flask with 4 l of dry toluene. The mixture was heated to 100°C and then exactly 2 l of dry N-methylpyrrolidinone were added. The mixture was heated to and then maintained at 115°C until the polymer dissolved (about 20 to 30 minutes). The solution was allowed to cool to 90°C and 4 l of hot toluene added in portions when the cooling solution appeared to change from a lustrous to a grainy appearance as precipitation of the polymer began. The polymer was separated by filtration under nitrogen, then dried in a vacuum oven tumble drier at 70°C for about 3 hours to start removal of solvent, then overnight at 90° to 100°C to substantially complete removal of N-methylpyrrolidinone. The product having an inherent viscosity of about 1.2, was stored in a dry box.

The thermal stability of this polymer was evaluated by melting the polymer in a rheometer at different temperatures and holding it at the temperature for a period. The following results were obtained (dashed lines indicate no sample was analyzed at that point):

**TABLE I**

| Temperature (°C) | Inherent Viscosity¹ | | | |
|---|---|---|---|---|
| | 10 | 20 | 30 | NT |
| 165 | 1.05 | 1.02 | 1.02 | ---- |
| 170 | 0.97 | 0.93 | 0.91 | 0.31 |
| 175 | 0.93 | 0.87 | 0.83 | ---- |
| 180 | 0.84 | 0.77 | 0.61 | ---- |
| 185 | 0.78 | 0.66 | ---- | ---- |
| 190 | 0.73 | ---- | ---- | ---- |

| | | | | |
|---|---|---|---|---|
| ¹ In deciliters/gram after the indicated number of minutes had elapsed. | | | | |

The column headed NT shows the result after heating for 10 minutes obtained from a sample of polymer which had not been treated with N-methylpyrrolidone.

These results show that the polymer treated with N-methylpyrrolidone was relatively stable at temperatures up to about 175°C. The polymer exhibited improved stability at all temperatures relative to the same polymer which had not been treated with N-methylpyrrolidone. The inherent viscosity after heating at 170°C for 10 minutes dropped 70.5 percent for the untreated sample but only 7.6 percent for the treated sample.

### Example 2

### Synthesis and treatment of poly[hexane-1,6-di(carbonyloxy)ethane-1,2-diamidocarbonylmethylene)] (i.e., PEA-6,2).

Exactly 333.00 g of 1,2-di(hydroxyacetamido)ethane were mixed well with 1.5 l of dry toluene in a 5 l flask. To this mixture was added about 1 l of toluene and then 398.98 g of distilled suberoyl chloride. The mixture was heated and stirred under nitrogen at 90°C for about 6 hours, then at reflux for about 2 hours. After refluxing for about 1.5 hours the inherent viscosity was about 1.1. After the 2 hours at reflux, the polymer was filtered and dried at 70°C in a vacuum tumble drier for about 2 hours. During drying for this period, the polymer became slightly gelled.

The dry polymer powder was placed in a 22 l flask with 2 l of dry toluene. The mixture was heated to 100°C and then exactly 2 l of dry N-methylpyrrolidinone were added. The mixture was heated to and then maintained at 115°C until the polymer dissolved (about 20 to 30 minutes). The solution was allowed to cool to room temperature and 4 l of hot toluene added in portions when the cooling solution appeared to change from a lustrous to a grainy appearance as precipitation of the polymer began. The polymer was separated by filtration under nitrogen, then dried in a vacuum oven tumble drier at 70°C for about 3 hours to start removal of solvent, then overnight at 90°C to substantially complete removal of N-methylpyrrolidinone. The product having an inherent viscosity of about 1.33 was stored in a dry box.

### Example 3

### Synthesis and treatment of poly[tetradecane-1,14-di(carbonyloxy)ethane-1,2-di(amidocarbonylmethylene)] (i.e., PEA-14,2).

Exactly 4.89 g of 1,2-di(hydroxyacetamido)ethane was mixed well with 0.05 l of dry toluene and placed in a large flask. To this mixture was added 8.975 g of distilled hexadecanedioyl chloride. The mixture was heated and stirred under nitrogen at 70°C for two hours and then at 90°C for about two hours, then at reflux for about 0.75 hour. To this reaction mixture was added 0.080 l of dry N-methylpyrrolidone. The polymer dissolved (20 to 30 minutes). To the solution was then added hot toluene with mixing to cause precipitation of the polymer. The white polymer was separated by filtration under nitrogen, then dried on a tray overnight in a vacuum oven at 80°C to remove toluene and N-methylpyrrolidone.

The resultant polymer was stored in a dry box. It was not soluble in 2,2,2-trifluoroethanol due to increased hydrocarbon content therefore its inherent viscosity could not be determined by that method. Based on its smooth, uniform appearance and the lack of lumps, it is believed that the polymer did not gel during synthesis. Filaments pulled from a melted sample of polymer were easily cold drawn by hand to yield tenacious fibers indicating that a satisfactory inherent viscosity (and therefore molecular weight) had been obtained.

### Comparative Example A

A poly(esteramide) produced as described in Example 5 of U.S. Patent No. 4,343,931 (i.e., PEA-2,12) was evaluated.

The relative absorption rates of certain poly(esteramides) of the invention and of Comparative Example A were evaluated by immersing 200 milligram filaments (formed in a rheometer by extrusion at 170°C) of the indicated polymer in 25 milliliters of pH 11 phosphate buffer held at 80°C for the indicated time to induce hydrolysis. The remaining weight of each sample was measured periodically and the results obtained, tabulated as percent of initial weight, were as follows (dashed lines indicate no sample was analyzed at that point):

**Table II**

| Time¹ | Example | | | |
|---|---|---|---|---|
| | 1 | 2 | 3 | A |
| 0 | 100 | 100 | 100 | 100 |
| 15 | ---- | 55 | ---- | ---- |
| 30 | ---- | 48 | ---- | ---- |
| 60 | 57 | 10 | ---- | 90 |
| 90 | ---- | 0 | ---- | ---- |
| 120 | 51 | ---- | 78.5 | 75 |
| 180 | 46 | ---- | ---- | 65 |
| 240 | 16 | ---- | 70 | 55 |
| 300 | 6 | ---- | ---- | 40 |
| 360 | 0 | ---- | 29.5 | 20² |
| 480 | ---- | ---- | 13 | 20 |
| 600 | ---- | ---- | 3 | 20 |

| | | | | |
|---|---|---|---|---|
| ¹ Elapsed residence time in minutes | | | | |
| ² Residual due to insoluble amidediol | | | | |

### Example 4

### Synthesis and treatment of poly[decane-1,10-di(carbonyloxy)ethane-1,2-di(amidocarbonylmethylene)]

Exactly 500.00 g of 1,2-di(hydroxyacetoamido)ethane were mixed well with 1.5 l of dry toluene and placed in a 22 l flask. To this mixture was added 3.5 l of toluene and then 758.29 g of distilled dodecanedioyl chloride. The mixture was heated and stirred under nitrogen at 90°C for 6 hours, then at reflux until the inherent viscosity of the polymer was 1.2 (about 2 hours). About 1.5 l of hot toluene were then added and mixed. Then 1.3 l of dry N-methylpyrrolidone and 1.3 l of dry N,N-dimethylformamide were added while stirring. The mixture was heated to and held at 115°C until the polymer had completely dissolved (taking about 20 to 30 minutes). The solution was allowed to cool to 90°C and 4 l of hot toluene were added in portions when the solution appeared to change from lustrous to grainy appearance as precipitation of polymer began. The slurry was then heated at 100°C and mixed for 15 minutes before filtration under nitrogen, then the separated polymer was tumble dried in a vacuum oven tumble drier at 70°C for about 3 hours to start removal of solvent, then dried overnight at 90° to 100°C to substantially complete removal of the amide group-containing solvents.

The resultant product, a white powder, had an inherent viscosity of about 1.16 and was stored in a dry box. After melting at 170°C, the product had an inherent viscosity of 1.12 and appeared to be whiter than the polymer of Example 1 after melting.

### Example 5

### Synthesis and treatment of poly(esteramide) reaction product derived from multiple acid dichlorides (i.e., a copolymerized mixture of PEA-10,2, PEA-8,2, PEA-6,2, and PEA-4,2).

To a mixture of 20.00 g of 1,2-di(hydroxyacetamido)ethane in 200 milliliters ("ml") of dry toluene, 7.58 g of dodecanedioyl chloride, 6.00 g of suberoyl chloride, 6.79 g of sebacoyl chloride, and 5.20 g of adipoyl chloride were added. The mixture was heated to 80°C and held at that temperature for 6 hours. The mixture was then heated to boiling and polymer began to agglomerate. To this hot mixture was added 100 ml of N-methylpyrrolidone in which the polymer dissolved. The mixture was heated to reflux for 2 hours. The polymer was then precipitated by the addition of hot toluene. After being allowed to precipitate, the polymer was separated from the mixture by filtration. It was then dried on a tray in a vacuum oven at 100°C.

The inherent viscosity was determined to be 0.85. Copolymerization such as the poly(esteramide) in this Example has been shown to reduce the crystallinity of the polymer, thereby improving the flexibility of resultant fibers.

## Claims

1. A bioabsorbable poly(esteramide) polymer characterized in that said polymer comprises a plurality of units of the general formula: 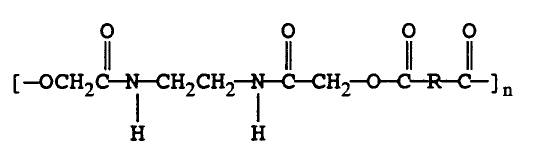 wherein:
a) R is selected from the group consisting of linear or branched alkylenes having from 2 to 25 carbon atoms; and
b) n has an average value between about 20 and about 1000.

2. The poly(esteramide) of claim 1 further characterized in that n has an average value between about 100 and about 400.

3. The poly(esteramide) of claim 1 further characterized in at least one of the following:
a) R is a linear alkylene having from 2 to 14 carbon atoms; or
b) R is a linear alkylene having from 4 to 12 carbon atoms; or
c) R is a linear alkylene having 10 carbon atoms; or
d) R is a linear alkylene having 6 carbon atoms.

4. The poly(esteramide) of claim 1 further characterized in at least one of the following:
a) said poly(esteramide) is a copolymer with R being independently alkylenes of different lengths selected from the class of alkylenes having 2 to 14 carbon atoms; or
b) said poly(esteramide) is a copolymer with R being independently alkylenes of different lengths selected from the class of alkylenes having 6 and 10 carbon atoms.

5. The poly(esteramide) of claim 1 further characterized in that prior to thermal processing, the inherent viscosity of said poly(esteramide) at 30°C in 2,2,2-trifluoroethanol is at least 1.0.

6. The poly(esteramide) of claim 1 characterized in that said polymer is prepared by:
a) reacting ethylenediamine with glycolic acid to form a diamidediol; and
b) reacting said diamidediol with one or more diacid chlorides having from 2 to 25 carbon atoms;
to yield said poly(esteramide) polymer.

7. An implantable bioabsorbable surgical device made from the polymer of claim 1.

8. The device of claim 7 further characterized in that said device comprises a fiber made of said polymer.

9. The device of claim 1 further characterized in that said device is a surgical suture or a ligament augmentation device.

10. A method for making poly(esteramide) polymer comprising:
a) reacting ethylenediamine with glycolic acid to form a diamidediol; and
b) reacting said diamidediol with one or more diacid chlorides having from 2 to 25 carbon atoms;
to yield said poly(esteramide) polymer.

## Patentansprüche

1. Biologisch absorbierbares Poly(esteramid)polymer, dadurch gekennzeichnet, daß das Polymer eine Mehrzahl von Einheiten der allgemeinen Formel: 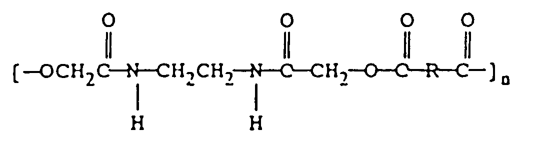 umfaßt, wobei:
a) R aus der aus linearen oder verzweigten Alkylenen mit 2 bis 25 Kohlenstoffatomen bestehenden Gruppe ausgewählt ist; und
b) n einen Mittelwert zwischen etwa 20 und etwa 1000 aufweist.

2. Poly(esteramid) nach Anspruch 1, weiterhin dadurch gekennzeichnet, daß n einen Mittelwert zwischen etwa 100 und etwa 400 aufweist.

3. Poly(esteramid) nach Anspruch 1, weiterhin durch wenigstens eines des Folgenden gekennzeichnet:
a) R ist ein lineares Alkylen mit 2 bis 14 Kohlenstoffatomen, oder
b) R ist ein lineares Alkylen mit 4 bis 12 Kohlenstoffatomen, oder
c) R ist ein lineares Alkylen mit 10 Kohlenstoffatomen, oder
d) R ist ein lineares Alkylen mit 6 Kohlenstoffatomen.

4. Poly(esteramid) nach Anspruch 1, weiterhin durch wenigstens eines des Folgenden gekennzeichnet:
a) das Poly(esteramid) ist ein Copolymer, wobei R unabhängig Alkylene mit unterschiedlichen Längen, ausgewählt aus der Klasse aus Alkylenen mit 2 bis 14 Kohlenstoffatomen, darstellt; oder
b) das Poly(esteramid) ist ein Copolymer, wobei R unabhängig Alkylene mit verschiedenen Längen, ausgewählt aus der Klasse aus Alkylenen mit 6 und 10 Kohlenstoffatomen, darstellt.

5. Poly(esteramid) nach Anspruch 1, weiterhin dadurch gekennzeichnet, daß vor der thermischen Verarbeitung die logarithmische Viskosität des Poly(esteramids) bei 30 °C in 2,2,2-Trifluorethanol wenigstens 1,0 beträgt.

6. Poly(esteramid) nach Anspruch 1, dadurch gekennzeichnet, daß das Polymer hergestellt wird durch das:
a) Umsetzen von Ethylendiamin mit Glycolsäure, wodurch ein Diamiddiol gebildet wird; und das
b) Umsetzen des Diamiddiols mit einem oder mehreren Disäurechloriden mit 2 bis 25 Kohlenstoffatomen;
wodurch das Poly(esteramid)polymer erhalten wird.

7. Implantierbare, biologisch absorbierbare, chirurgische Vorrichtung, hergestellt aus dem Polymer nach Anspruch 1.

8. Vorrichtung nach Anspruch 7, weiterhin dadurch gekennzeichnet, daß die Vorrichtung eine aus dem Polymer hergestellte Faser umfaßt.

9. Vorrichtung nach Anspruch 1, weiterhin dadurch gekennzeichnet, daß es sich bei der Vorrichtung um ein Chirurgie-Nahtmaterial oder eine Vorrichtung zum Ligamentaufbau handelt.

10. Verfahren zur Herstellung von Poly(esteramid)polymer, umfassend das:
a) Umsetzen von Ethylendiamin mit Glycolsäure, wodurch ein Diamiddiol gebildet wird; und das
b) Umsetzen des Diamiddiols mit einem oder mehreren Disäurechloriden mit 2 bis 25 Kohlenstoffatomen;
wodurch das Poly(esteramid)polymer erhalten wird.

## Revendications

1. Polymère poly(esteramide) bioabsorbable caractérisé en ce que ledit polymère comprend une pluralité de motifs de formule générale : 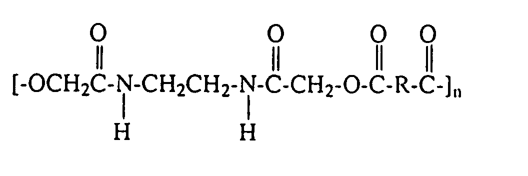 dans laquelle :
a) R est choisi dans le groupe formé par des alkylènes linéaires ou ramifiés ayant de 2 à 25 atomes de carbone ; et
b) n possède une valeur moyenne comprise entre environ 20 et environ 1 000.

2. Poly(esteramide) selon la revendication 1, caractérisé de plus en ce que n possède une valeur moyenne comprise entre environ 100 et environ 400.

3. Poly(esteramide) selon la revendication 1, caractérisé de plus par au moins un élément parmi ce qui suit :
a) R est un alkylène linéaire ayant de 2 à 14 atomes de carbone ; ou
b) R est un alkylène linéaire ayant de 4 à 12 atomes de carbone ; ou
c) R est un alkylène linéaire ayant 10 atomes de carbone ; ou
d) R est un alkylène linéaire ayant 6 atomes de carbone.

4. Poly(esteramide) selon la revendication 1, caractérisé de plus par au moins un élément parmi ce qui suit :
a) ledit poly(esteramide) est un copolymère, R étant indépendamment des alkylènes de différentes longueurs choisis dans la classe des alkylènes ayant 2 à 14 atomes de carbone ; ou
b) ledit poly(esteramide) est un copolymère, R étant indépendamment des alkylènes de différentes longueurs choisis dans la classe des alkylènes ayant 6 et 10 atomes de carbone.

5. Poly(esteramide) selon la revendication 1, caractérisé de plus en ce qu'avant un traitement thermique, la viscosité intrinsèque dudit poly(esteramide) à 30°C dans le 2,2,2-trifluoroéthanol est au moins de 1,0.

6. Poly(esteramide) selon la revendication 1, caractérisé en ce que ledit polymère est préparé :
a) en faisant réagir l'éthylènediamine avec l'acide glycolique pour former un diamidediol ; et
b) en faisant réagir ledit diamidediol avec un ou plusieurs chlorures de diacide ayant de 2 à 25 atomes de carbone ;
pour produire ledit polymère poly(esteramide).

7. Dispositif chirurgical bioabsorbable implantable fait à partir du polymère selon la revendication 1.

8. Dispositif selon la revendication 7, caractérisé de plus en ce que ledit dispositif comprend une fibre faite dudit polymère.

9. Dispositif selon la revendication 1, caractérisé de plus en ce que ledit dispositif est une suture chirurgicale ou un dispositif d'augmentation de ligament.

10. Procédé pour fabriquer un polymère poly(esteramide) comprenant les étapes consistant
a) à faire réagir l'éthylènediamine avec l'acide glycolique pour former un diamidediol ; et
b) à faire réagir ledit diamidediol avec un ou plusieurs chlorures de diacide ayant de 2 à 25 atomes de carbone ;
pour produire ledit polymère poly(esteramide).
